# EUROPEAN PATENT SPECIFICATION

(11) **EP 1 254 636 B1**
(45) Date of publication and mention of the grant of the patent: **13.10.2004**
(21) Application number: 02008131.1
(22) Date of filing: 11.04.2002
(51) Int. Cl.: A61B 17/072, A61B 17/32

(54) **Surgical instrument for stapling and/or cutting with a wavy tissue interface**
Chirurgisches Gerät zum klammern und/oder schneiden mit wellenförmiger Kontaktfläche
Instrument chirurgical servant à agrafer et/ou à couper avec une interface tissulaire ondulée

(30) Priority: 02.05.2001 DE 10121305
(43) Date of publication of application: 06.11.2002
(73) Proprietor: Ethicon Endo-Surgery (Europe) GmbH, 22851 Norderstedt (DE)
(72) Inventor: Cosaro, Santi, 22085 Hamburg (DE)
(74) Representative: UEXKÜLL & STOLBERG

(56) References cited:
- US-A- 3 771 526
- US-A- 4 527 724
- US-A- 5 307 976

## Description

The invention relates to a surgical instrument having a jaw assembly with a generally straight first tissue interface, a generally straight second tissue interface and a tissue effector. This instrument can be designed as a stapling instrument and/or a cutting instrument.

Instruments of this kind are widely used in surgery. Generally, the surgical instrument comprises a frame having a body portion and a handle and, in the distal region of the instrument, a jaw assembly. The jaw assembly has a generally straight design with a generally straight first tissue interface and a generally straight second tissue interface, the jaw assembly being adapted to grip tissue of a patient between the first tissue interface and the second tissue interface. Moreover, a tissue effector for penetrating the gripped tissue is provided. An example of a tissue effector is a set of staples which are arranged in the jaw assembly and, upon actuation of the instrument, exit from the first tissue interface and penetrate the tissue, until their ends are formed or bent by the second tissue interface. Another example is a knife for cutting the tissue when the instrument is actuated. The knife can be used in combination with the staples, but it can also be arranged in a pure cutting instrument.

U.S. patent 4 527 724 discloses a linear surgical stapling instrument in which a straight jaw assembly is located at the distal end of a shaft. The longitudinal axis of the jaw assembly extends transversely with respect to the longitudinal axis of the shaft.

U.S. Patent 4 633 874 describes a surgical stapling instrument in which the longitudinal axis of the jaw assembly coincides with the longitudinal axis of the rest of the instrument. A pusher bar and knife blade assembly is slidable longitudinally relative to the jaw members to sequentially drive staples from a cartridge and form the staples against one of the tissue interfaces (which is designed as an anvil) to produce a pair of laterally spaces rows of formed staples in tissue gripped between the jaw members (i.e. between the first tissue interface and the second tissue interface) and to cut the tissue along a line between the staple rows.

Another straight or linear stapling instrument in which the longitudinal axis of the jaw assembly runs along the longitudinal axis of the whole instrument is disclosed in U.S. patent 5 307 976. This document also describes details of the actuating components and safety means of the instrument.

In all these surgical instruments, the tissue interfaces, i.e. those parts of the jaw assembly which contact the patient's tissue to be effected (e.g., stapled and/or cut), are designed as generally flat surfaces. This holds for instruments used in open surgery and in endoscopic surgery. In straight (linear) cutters for open or endoscopic surgery, the tissue interfaces usually extend along the longitudinal axis of the instrument. For linear staplers applied in open surgery, the tissue interfaces usually are arranged transversally with respect to the longitudinal axis of the instrument. In the current designs of these kinds of surgical instruments, the jaw assembly is generally longer than the length of tissue which can be gripped between the first tissue interface and the second tissue interface. This can make it difficult to manipulate and utilize the instrument in certain situations, in particular in space-limited anatomical regions.

It is the object of the invention to provide a surgical instrument of the above-described kind which is able to effect tissue in a more space-saving manner.

This problem is solved by a surgical instrument having the features of claim 1. Advantageous embodiments of the invention follow from the dependent claims.

The surgical instrument according to the invention comprises a frame having a body portion and a handle, as well as a jaw assembly in the distal region of the instrument. The jaw assembly has a generally straight first tissue interface, a generally straight second tissue interface and a tissue effector, and the jaw assembly is adapted to grip tissue of a patient between the first tissue interface and the second tissue interface and to penetrate the gripped tissue by the tissue effector. A moving device is adapted to move the first tissue interface relative to the second tissue interface. An actuating device is adapted to actuate the tissue effector. According to the invention, the first tissue interface and the second tissue interface have a generally wavy shape, locally and superimposed to their general straightness. The crests and valleys of the first tissue interface generally match the valleys and crests, respectively, of the second tissue interface.

In other words, the tissue interfaces are not flat, as in conventional instruments, but have a three-dimensional, wavy shape along the length of the jaw assembly, e.g. a sinusoidal shape. Thus, the path length following the valleys and crests of the wavy shape is greater than the linear extension of the tissue interfaces. Generally, this allows the instrument to effect a length of tissue greater than the length of the jaw assembly. If the surgical instrument is a stapling instrument and/or a cutting instrument, a staple line and/or cutting line can be created which follows the shape of the tissue gripped between the first tissue interface and the second tissue interface, i.e. the generally wavy shape of the tissue interfaces, and which is effectively longer than the staple line or the cutting line of a conventional instrument. The greater length of the staple line or cutting line has advantages, e.g., for a given length of a staple line or cutting line, the jaw assembly of the surgical instrument according to the invention can be smaller than the jaw assembly of a conventional instrument. Thus, the invention allows for the design of smaller or shorter and less bulky jaw assembly components that will facilitate easier access to anatomic sites.

As already mentioned, the surgical instrument can be a stapling instrument. In this case, the jaw assembly includes a cartridge device having the first tissue interface and an anvil having the second tissue interface. The tissue effector comprises, associated to the cartridge device, at least one generally straight row of staples which, upon actuation of the actuating device, exit from the first tissue interface, the second tissue interface being adapted to form the ends of the staples. Preferably, the line defining the row of staples follows the wavy shape of the first tissue interface. Alternatively, e.g., the staples could be arranged at the top of the crests and/or at the bottom of the valleys of the first tissue interface.

In an advantageous version of a stapling instrument, the tissue effector comprises a knife, which is contained in the cartridge device, runs generally in parallel to the row of staples and, upon actuation of the actuating device, moves towards the anvil. The knife can have a wavy cutting edge, which, preferably, generally runs in parallel to the wavy shape of the first tissue interface.

In another advantageous version of a stapling instrument, the tissue effector comprises a knife, which is adapted to move, upon actuation of the actuating device, from one end region of the cartridge device to the opposite end region of the cartridge device, generally in parallel to and along the row of staples, in order to sequentially cut the tissue gripped between the first tissue interface and the second tissue interface.

Preferably, in a stapling instrument comprising a knife, there is at least one row of staples on each side of the knife. This allows opposing layers of tissue to be connected at both sides of the knife, i.e. to create two closed tissue parts which are separated when the knife is actuated.

In advantageous versions of a stapling instrument, the anvil is removable, and the cartridge device comprises a removable cartridge containing the staples.

The surgical instrument according to the invention can also be designed as a cutting instrument (without stapling function). In this case, the jaw assembly includes a first gripping jaw having the first tissue interface and a second gripping jaw having the second tissue interface. The tissue effector comprises a knife, which is adapted to cut, upon actuation of the actuating device, the tissue gripped between the first tissue interface and the second tissue interface.

In a preferred version of a cutting instrument, the knife is generally straight, generally extends along the jaw assembly, and, upon actuation of the actuating device, moves from the first tissue interface towards the second tissue interface. In this case, the knife can have a wavy cutting edge, which, preferably, generally runs in parallel to the wavy shape of the first tissue interface.

In another design of a cutting instrument, the knife is adapted to move, upon actuation of the actuating device, from one end region of the gripping jaws to the opposite end region of the gripping jaws, generally along the gripping jaws, in order to sequentially cut the tissue gripped between the first tissue interface and the second tissue interface.

In advantageous versions of a stapling instrument and/or a cutting instrument, the moving device is adapted to.move the first tissue interface relative to the second tissue interface in a generally parallel relationship. This design facilitates insertion of the tissue into the space between the first tissue interface and the second tissue interface when these interfaces are in the spaced apart state.

Preferably, the body portion of the frame of the surgical instrument includes a shaft. This allows for two basic geometries of the instrument. In one of these geometries, the longitudinal axes of the first tissue interface and the second tissue interface run transversally with respect to the longitudinal axis of the shaft. This design is advantageous for applications in open surgery. When the instrument includes a knife, a generally straight knife which generally extends along the jaw assembly allows for a mechanically simple actuating device. In the other basic geometry, the longitudinal axes of the first tissue interface and the second tissue interface run generally in parallel with respect to the longitudinal axis of the shaft. This geometry is advantageous for endoscopic applications, and a sequentially cutting knife generally involves a simple design of the actuating device.

The jaw assembly can be removably mounted in the distal end region of the body portion. This allows for a design of the surgical instrument in which the jaw assembly is disposable, whereas the rest of the instrument, which is not contaminated or only slightly contaminated in the surgical procedure and which includes many components of the moving device and the actuating device, can be re-used after sterilization.

Apart from the three-dimensional shape of the tissue interfaces and any adaptions caused thereby, the surgical instrument according to the invention is generally designed as a conventional instrument. That means, it includes components like a handle, a shaft, gripping jaws or an anvil and a cartridge device, mechanical members of the moving device and the actuating device, safety elements like locking means against unintentional operation, and so on, which are generally known from the prior art, e.g., U.S. patents 4 527 724, 4 633 874, or 5 307 976. Moreover, the surgical instrument according to the invention can be used in a similar way as the prior art instruments.

In the following, the surgical instrument according to the invention is further explained by means of embodiments. The drawings show in
- Figure 1: a side view of a first embodiment of the surgical instrument according to the invention,
- Figure 2: a magnified side view of the jaw assembly of the instrument of Figure 1,
- Figure 3: a side view of a second embodiment of the surgical instrument according to the invention,
- Figure 4: a side view of a third embodiment of the surgical instrument according to the invention, and
- Figure 5: a magnified side view of the jaw assembly of the instrument of Figure 4.

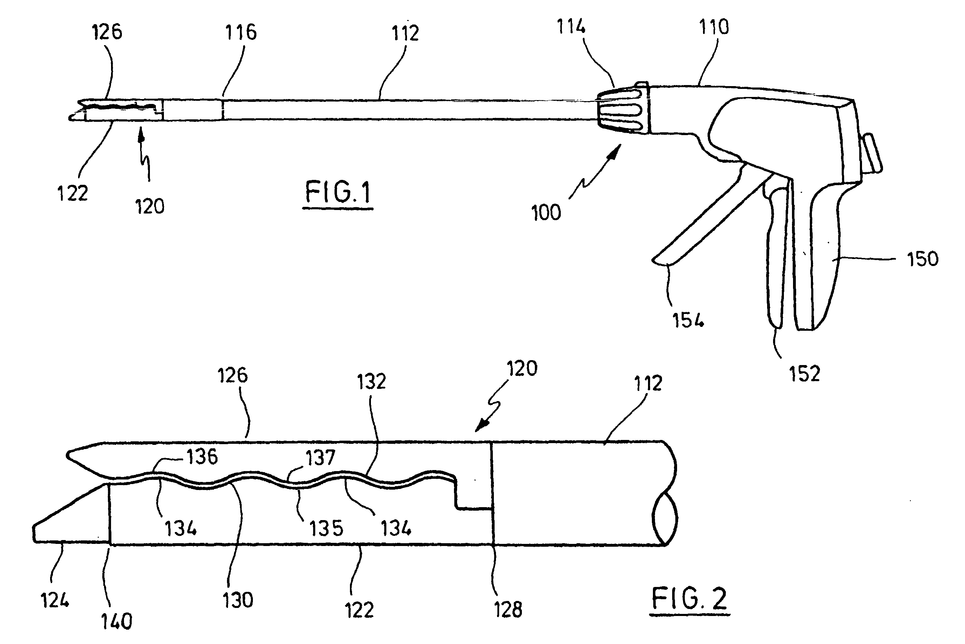

Figure 1 illustrates a surgical instrument 100 which is designed as a linear stapler and can be used in endoscopic applications. This general type of instrument is known, e.g. from U.S. patent 5 307 976.

The instrument 100 comprises a handle section 110 and a shaft 112, which is rotatably connected to the handle section 110 via rotating means 114. The handle section 110 and the shaft 112 form a body portion.

At the distal end 116 of the shaft 112, a jaw assembly 120 is mounted in a removable manner. Figure 2 is a magnified view of the jaw assembly 120. The jaw assembly 120 includes a cartridge device 122 (lower jaw in Figures 1 and 2) and an anvil 126 (upper jaw in Figures 1 and 2). The anvil 126 is swivellably mounted in the jaw assembly 120, but when the anvil 126 is close to the cartridge device 122, it moves in a parallel relationship with respect to the cartridge device 122.

The cartridge device 122 comprises a removable cartridge 124, which essentially extends along the length of the cartridge device 122. Figure 2 shows the distal end region of the cartridge 124 which protrudes from a mounting base provided in the cartridge device 122. In the embodiment, the cartridge 124 contains six parallel straight rows of staples in which staples of adjacent rows are staggered with respect to each other. The ends of the staples point towards the anvil 126. The surface of the cartridge device 122 including the cartridge 124 opposite to the anvil 126 is called first tissue interface 130.

Similarly, the surface of the anvil 126 opposite to the cartridge device 122 is called second tissue interface 132. The second tissue interface comprises six parallel rows of depressions, which match to the positions of the staples in the cartridge 124 and are designed to form the pointed ends of the staples when they are expelled from the cartridge 124 upon actuation of the instrument 100.

The features of the instrument 100 described so far are those of a conventional stapler, and further details of the embodiment can be found in U.S. patent 5 307 976.

Whereas in a conventional stapler the first tissue interface and the second tissue interface are essentially flat or planar, the first tissue interface 130 of the instrument 100 has a generally wavy shape with crests 134 and valleys 135, see Figure 2. The second tissue interface 132 has a generally wavy shape as well, and its valleys 136 and crests 137 match to the crests 134 and valleys 135, respectively, of the first tissue interface 130, see Figure 2.

The proximal area of the instrument 100 is designed in a conventional manner, see U.S. patent 5 307 976 for more details on the embodiment. In summary, a handle 150 emerging from the handle section 110 serves for holding the instrument 100. A lever 152 is a component of a moving device for moving the first tissue interface 130 relative to the second tissue interface 132. That means, when the lever 152 is swivelled towards the handle 150, the anvil 126 moves towards the cartridge device 122 (i.e., the jaws of the jaw assembly 120 close), the force being transmitted by further components in the handle section 110, the shaft 112 and the jaw assembly 120, as explained, e.g., in U.S. patent 5 307 976.

A firing trigger 154 is actuated in order to expel the staples from the cartridge 124 towards the anvil 126 and, at the same time, to move a knife (not shown in the figures) from the proximal end region 128 to the distal end region 140 of the cartridge device 122. This knife is contained in the cartridge device 122 and runs in parallel to the rows of staples, wherein, in the embodiment, there are three rows of staples on each side of the knife. Again, further details of the knife and the actuating mechanism are given in U.S. patent 5 307 976.

The instrument 100 is used in a similar way as a conventional stapler. Tissue parts to be stapled (and cut) are introduced between the first tissue interface 130 and the second tissue interface 132 when the anvil 126 is moved away from the cartridge device 122. Then the anvil 126 is closed by moving it towards the cartridge device 122 via the lever 152, thus gripping and clamping the tissue between the first tissue interface 130 and the second tissue interface 132. In contrast to a conventional stapler, the tissue does not remain flat, but adapts to the wavy shape of the first tissue interface 130 and the second tissue interface 132. In this way, the tissue length effected by the jaw assembly 120 can be longer than the linear length of the tissue interfaces 130, 132. This allows the manipulation of a given length of tissue by an effectively smaller jaw assembly 120.

When the firing trigger 154 is actuated, the staples are expelled from the cartridge 124, penetrate the tissue and are formed by means of the anvil 126. Almost simultaneously the knife moves from the proximal end region 128 to the distal end region 140 of the cartridge device 122 and sequentially cuts the tissue along a line in-between the rows of staples.

The staple expelling mechanism disclosed in U.S. patent 5 307 976 drives the staples in a direction which is essentially perpendicular with respect to the longitudinal axis of the jaw assembly. The same type of mechanism could be used in the instrument 100. If it is preferred, however, that the staples are driven perpendicularly with respect to the local slope of the wavy shape of the first tissue interface 130, the mechanism has to be modified somewhat.

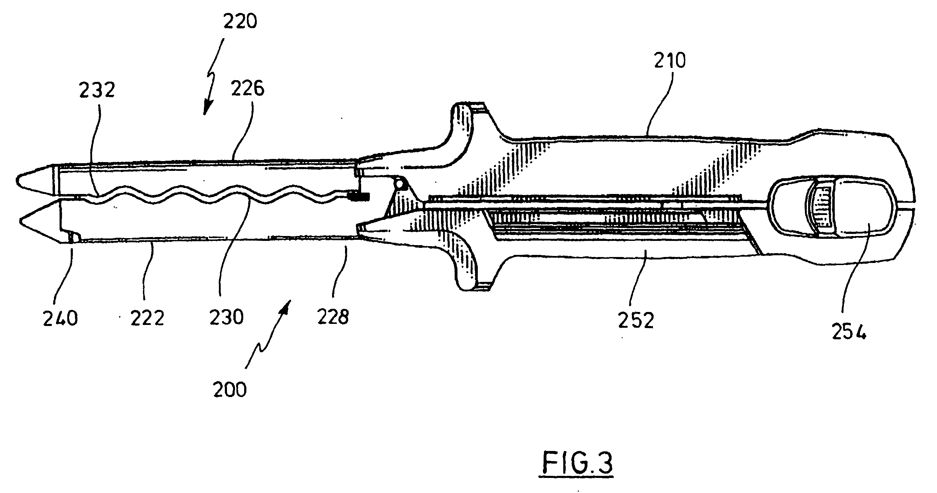

Figure 3 shows an embodiment of another surgical stapling instrument 200. The design of the instrument 200 is similar to that of the stapling instrument disclosed in U.S. patent 4 633 874.

The instrument 200 comprises a body portion 210 with integrated handle and a jaw assembly 220. Similar to the instrument 100 described above, the jaw assembly 220 includes a cartridge device 222, which comprises a first tissue interface 230, and an anvil 226, which comprises a second tissue interface 232. In the embodiment, the cartridge device 222 contains four parallel straight rows of staples and a knife running along the center line of the staple arrangement for sequentially cutting tissue gripped between the cartridge device 222 and the anvil 226.

The first tissue interface 230 and the second tissue interface 232 have a wavy shape, like in the instrument 100.

To operate the instrument 200, the anvil 226 is moved towards the cartridge device 222 in a parallel relationship by means of a lever 252 in order to grip or clamp tissue between the cartridge device 222 and the anvil 226. In the state shown in Figure 3, the lever 252 is swivelled in a position parallel to the body portion 210. Then an actuator 254 is pushed along the body portion 210 in distal direction, which causes the staples to be expelled from the cartridge device 222 for penetrating the tissue, the pointed ends of the staples being bent by the anvil 226, and to move the knife from the proximal end region 228 to the distal end region 240 of the cartridge device 222 for sequentially cutting the tissue.

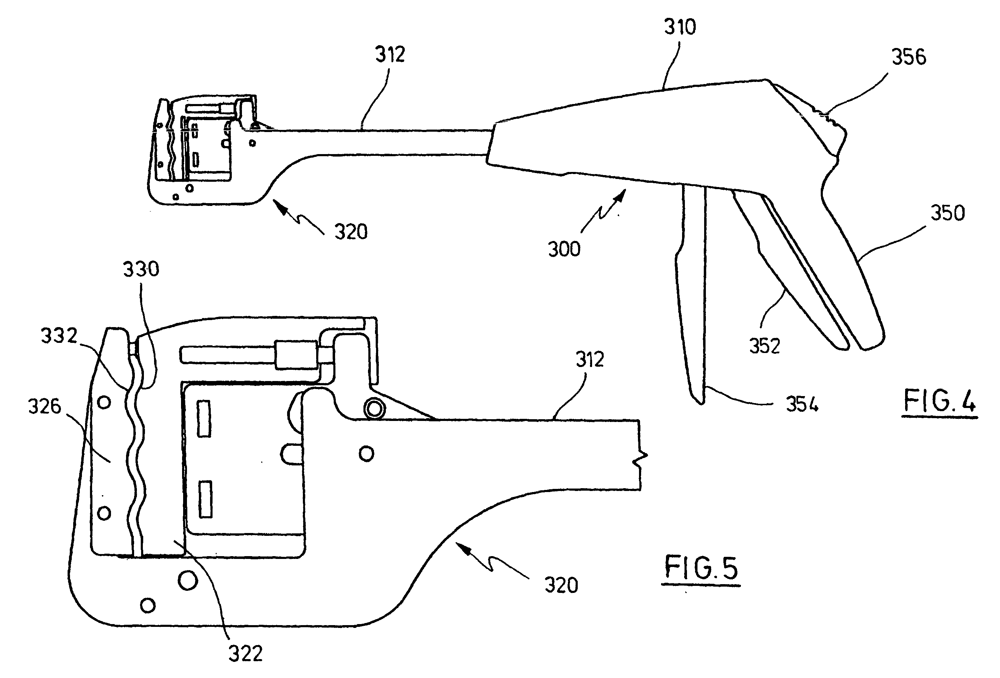

Figure 4 is an overall view of a surgical instrument 300 according to a third embodiment. The instrument 300 includes a handle section 310, a shaft 312 and a jaw assembly 320 at the distal end of the shaft 312. Figure 5 is a magnified view of the jaw assembly 320.

The jaw assembly 320 comprises a cartridge device 322 and an anvil 326. Whereas in the instruments 100 and 200, the respective cartridge device and anvil are aligned in parallel to the longitudinal axis of the instrument, the anvil 326 and the cartridge device 322 extend transversally with respect to the shaft 312. The instrument 300 is designed for open surgery. The anvil 326 is rigidly connected to an extension of shaft 312, and the cartridge device 322 can be moved towards and away from the anvil 326, while a parallel relationship between a first tissue interface 330 provided at the cartridge device 322 and a second tissue interface 332 provided at, the anvil 326 is maintained.

As in the previous embodiments, the first tissue interface 330 and the second tissue interface 332 have a wavy shape, in contrast to comparable instruments of the prior art. An example of such prior art instrument is described in U.S. patent 4 527 724 which also discloses details on the actuating elements of the instrument.

As before, the cartridge device 322 includes one or more parallel straight rows of staples, and the anvil 326 is adapted to form the ends of the staples when they are driven out of the cartridge device, here essentially simultaneously in the distal direction.

Moreover, the cartridge device 322 contains a knife which extends over the width of the cartridge device 322 and is guided between two rows of staples. The knife can be moved towards the anvil 326 in order to cut the tissue gripped or clamped between the anvil 326 and the cartridge device 322 essentially in one instant. In the embodiment, the knife has a wavy cutting edge which runs in parallel to the wavy shape of the first tissue interface 330.

In its proximal end region, the instrument 300 comprises a handle 350, a lever 352, a firing trigger 354, and a safety button 356. When the lever 352 is pulled towards the handle 350, the cartridge device 322 moves towards the anvil 326. In order to actuate the instrument 300, the firing trigger 354 is pulled towards the handle 350. This causes the staples and the knife to be driven towards the anvil 326 and to penetrate the tissue gripped in-between the first tissue interface 330 and the second tissue interface 332 in order to staple and cut the tissue.

Each of the instruments 100, 200, and 300 is designed as a stapler/cutter for stapling and cutting tissue. The invention, however, also includes pure stapling instruments (without a knife or cutter) and pure cutting instruments (without stapling means). In a pure cutting instrument, the jaw assembly includes gripping jaws having wavy-shaped tissue interfaces and a knife.

## Claims

1. A surgical instrument, comprising
- a frame having a body portion (110, 112; 210; 310, 312) and a handle (150; 210; 350),
- a jaw assembly (120; 220; 320) in the distal region of the instrument (100; 200; 300), the jaw assembly (120; 220; 320) having a generally straight first tissue interface (130; 230; 330), a generally straight second tissue interface (132; 232; 332) and a tissue effector, and the jaw assembly (120; 220; 320) being adapted to grip tissue of a patient between the first tissue interface (130; 230; 330) and the second tissue interface (132; 232; 332) and to penetrate the gripped tissue by the tissue effector,
- a moving device (152; 252; 352) adapted to move the first tissue interface (130; 230; 330) relative to the second tissue interface (132; 232; 332), and
- an actuating device (154; 254; 354) adapted to actuate the tissue effector,
- **characterized in that** both the first tissue interface (130; 230; 330) and the second tissue interface (132; 232; 332), locally and superimposed to their general straightness, have a generally wavy shape, the crests (134) and valleys (135) of the first tissue interface (130; 230; 330) generally matching the valleys (136) and crests (137), respectively, of the second tissue interface (132; 232; 332).

2. Surgical instrument according to claim 1, **characterized in that** the surgical instrument (100; 200; 300) is a stapling instrument, wherein the jaw assembly (120; 220; 320) includes a cartridge device (122; 222; 322) having the first tissue interface (130; 230; 330) and an anvil (126; 226; 326) having the second tissue interface (132; 232; 332), the tissue effector comprising, associated to the cartridge device (122; 222; 322), at least one generally straight row of staples which, upon actuation of the actuating device (154; 254; 354), exit from the first tissue interface (130; 230; 330), the second tissue interface (132; 232; 332) being adapted to form the ends of the staples.

3. Surgical instrument according to claim 2, **characterized in that** the line defining the row of staples follows the wavy shape of the first tissue interface (130; 230; 330).

4. Surgical instrument according to claim 2 or 3, **characterized in that** the tissue effector comprises a knife, which is contained in the cartridge device (122; 222; 322), runs generally in parallel to the row of staples and, upon actuation of the actuating device (154; 254; 354), moves towards the anvil (126; 226; 326).

5. Surgical instrument according to claim 4, **characterized in that** the knife has a wavy cutting edge, which, preferably, generally runs in parallel to the wavy shape of the first tissue interface (330).

6. Surgical instrument according to claim 2 or 3, **characterized in that** the tissue effector comprises a knife, which is adapted to move, upon actuation of the actuating device (154; 254), from one end region (128; 228) of the cartridge device (122; 222) to the opposite end region (140; 240) of the cartridge device (122; 222), generally in parallel to and along the row of staples, in order to sequentially cut the tissue gripped between the first tissue interface (130; 230) and the second tissue interface (132; 232).

7. Surgical instrument according to one of claims 4 to 6, **characterized in that** there is at least one row of staples on each side of the knife.

8. Surgical instrument according to one of claims 2 to 7, **characterized in that** the anvil is removable.

9. Surgical instrument according to one of claims 2 to 8, **characterized in that** the cartridge device (122; 222; 322) comprises a removable cartridge (124) containing the staples.

10. Surgical instrument according to claim 1, **characterized in that** the surgical instrument is a cutting instrument, wherein the jaw assembly includes a first gripping jaw having the first tissue interface and a second gripping jaw having the second tissue interface, the tissue effector comprising a knife, which is adapted to cut, upon actuation of the actuating device, the tissue gripped between the first tissue interface and the second tissue interface.

11. Surgical instrument according to claim 10, **characterized in that** the knife is generally straight, generally extends along the jaw assembly, and, upon actuation of the actuating device, moves from the first tissue interface towards the second tissue interface.

12. Surgical instrument according to claim 11, **characterized in that** the knife has a wavy cutting edge, which, preferably, generally runs in parallel to the wavy shape of the first tissue interface.

13. Surgical instrument according to claim 10, **characterized in that** the knife is adapted to move, upon actuation of the actuating device, from one end region of the gripping jaws to the opposite end region of the gripping jaws, generally along the gripping jaws, in order to sequentially cut the tissue gripped between the first tissue interface and the second tissue interface.

14. Surgical instrument according to one of claims 1 to 13, **characterized in that** the moving device (152; 252; 352) is adapted to move the first tissue interface (130; 230; 330) relative to the second tissue interface (132; 232; 332) in a generally parallel relationship.

15. Surgical instrument according to one of claims 1 to 14, **characterized in that** the body portion (310, 312) of the frame includes a shaft (312) and **in that** the longitudinal axes of the first tissue interface (330) and the second tissue interface (332) run transversely with respect to the longitudinal axis of the shaft (312).

16. Surgical instrument according to one of claims 1 to 14, **characterized in that** the body portion (110, 112; 210) of the frame includes a shaft (112; 210) and **in that** the longitudinal axes of the first tissue interface (130; 230) and the second tissue interface (132; 232) run generally in parallel with respect to the longitudinal axis of the shaft (112; 210).

17. Surgical instrument according to one of claims 1 to 16, **characterized in that** the surgical instrument is an endoscopic instrument (100).

18. Surgical instrument according to one of claims 1 to 17, **characterized in that** the jaw assembly (120) is removably mounted in the distal end region (116) of the body portion (110; 112).

## Patentansprüche

1. Chirurgisches Instrument mit
- einem Rahmen mit einem Körperbereich (110, 112; 210; 310, 312) und einem Handgriff (150; 210; 350),
- einer Backenanordnung (120; 220; 320) im distalen Bereich des Instruments (100; 200; 300), wobei die Backenanordnung (120; 220; 320) eine allgemein gerade erste Gewebe-Grenzfläche (130; 230; 330), eine allgemein gerade zweite Gewebe-Grenzfläche (132; 232; 332) und einen Gewebe-Effektor hat, und wobei die Backenanordnung (120; 220; 320) dazu eingerichtet ist, Gewebe eines Patienten zwischen der ersten Gewebe-Grenzfläche (130; 230; 330) und der zweiten Gewebe-Grenzfläche (132; 232; 332) zu greifen und das gegriffene Gewebe mit dem Gewebe-Effektor zu durchdringen,
- einer Bewegungseinrichtung (152; 252; 352), die dazu eingerichtet ist, die erste Gewebe-Grenzfläche (130; 230; 330) relativ zu der zweiten Gewebe-Grenzfläche (132; 232; 332) zu bewegen und
- einer Betätigungseinrichtung (154; 254; 354), die dazu eingerichtet ist, den Gewebe-Effektor zu betätigen,
- **dadurch gekennzeichnet, dass** sowohl die erste Gewebe-Grenzfläche (130; 230; 330) als auch die zweite Gewebe-Grenzfläche (132; 232; 332), lokal und ihrer allgemeinen Geradheit überlagert, eine allgemein wellenförmige Form haben, wobei die Berge (134) und Täler (135) der ersten Gewebe-Grenzfläche (130; 230; 330) allgemein zu den Tälern (136) bzw Bergen (137) der zweiten Gewebe-Grenzfläche (132; 232; 332) passen.

2. Chirurgisches Instrument nach Anspruch 1, **dadurch gekennzeichnet, dass** das chirurgische Instrument (100; 200; 300) ein Klammerinstrument ist, wobei die Backenanordnung (120; 220; 320) eine Patroneneinrichtung (122; 222; 322) mit der ersten Gewebe-Grenzfläche (130; 230; 330) und ein Gegenlager (126; 226 326) mit der zweiten Gewebe-Grenzfläche (132; 232; 332) enthält, wobei der Gewebe-Effektor, verknüpft mit der Patrconeneinrichtung (122; 222; 322), wenigstens eine allgemein gerade Reihe von Klammern aufweist, die bei Betätigung der Betätigungseinrichtung (154; 254; 354) von der ersten Gewebe-Grenzfläche (130; 230; 330) ausgehen, wobei die zweite Gewebe-Grenzfläche (132; 232; 332) dazu eingerichtet ist, die Enden der Klammern zu formen.

3. Chirurgisches Instrument nach Anspruch 2, **dadurch gekennzeichnet, dass** die die Klammerreihe definierende Linie der wellenförmigen Form der ersten Gewebe-Grenzfläche (130; 230; 330) folgt.

4. Chirurgisches Instrument nach Anspruch 2 oder 3, **dadurch gekennzeichnet, dass** der Gewebe-Effektor ein Messer aufweist, das in der Patronenanordnung (122; 222; 322) enthalten ist, allgemein parallel zu der Klammerreihe verläuft und sich bei Betätigung der Betätigungseinrichtung (154; 254; 354) auf das Gegenlager (126; 226; 326) zu bewegt.

5. Chirurgisches Instrument nach Anspruch 4, **dadurch gekennzeichnet, dass** das Messer eine wellenförmige Schneidkante hat, die vorzugsweise allgemein parallel zu der wellenförmigen Form der ersten Gewebe-Grenzfläche (330) verläuft.

6. Chirurgisches Instrument nach Anspruch 2 oder 3, **dadurch gekennzeichnet, dass** der Gewebe-Effektor ein Messer aufweist, das dazu eingerichtet ist, sich bei Betätigung der Betätigungseinrichtung (154; 254) von einem Endbereich (128; 228) der Patroneneinrichtung (122; 222) zu dem gegenüberliegenden Endbereich (140; 240) der Patroneneinrichtung (122; 222) zu bewegen, allgemein parallel zu und entlang der Klammerreihe, um das zwischen der ersten Gewebe-Grenzfläche (130; 230) und der zweiten Gewebe-Grenzfläche (132; 232) gegriffene Gewebe sequentiell zu schneiden.

7. Chirurgisches Instrument nach einem der Ansprüche 4 bis 6, **dadurch gekennzeichnet, dass** auf jeder Seite des Messers wenigstens eine Klammerreihe vorgesehen ist.

8. Chirurgisches Instrument nach einem der Ansprüche 2 bis 7, **dadurch gekennzeichnet, dass** das Gegenlager entfernbar ist.

9. Chirurgisches Instrument nach einem der Ansprüche 2 bis 8, **dadurch gekennzeichnet, dass** die Patroneneinrichtung (122; 222; 322) eine entfernbare Patrone (124) aufweist, die die Klammern enthält.

10. Chirurgisches Instrument nach Anspruch 1, **dadurch gekennzeichnet, dass** das chirurgische Instrument ein Schneidinstrument ist, wobei die Backenanordnung eine erste Greifbacke mit der ersten Gewebe-Grenzfläche und eine zweite Greifbacke mit der zweiten Gewebe-Grenzfläche enthält, wobei der Gewebe-Effektor ein Messer aufweist, das dazu eingerichtet ist, bei Betätigung der Betätigungseinrichtung das zwischen der ersten Gewebe-Grenzfläche und der zweiten Gewebe-Grenzfläche gegriffene Gewebe zu schneiden.

11. Chirurgisches Instrument nach Anspruch 10, **dadurch gekennzeichnet, dass** das Messer allgemein gerade ist, sich allgemein entlang der Backenanordnung erstreckt und sich bei Betätigung der Betätigungseinrichtung von der ersten Gewebe-Grenzfläche auf die zweite Gewebe-Grenzfläche zu bewegt.

12. Chirurgisches Instrument nach Anspruch 11, **dadurch gekennzeichnet, dass** das Messer eine wellenförmige Schneidkante hat, die vorzugsweise allgemein parallel zu der wellenförmigen Form der ersten Gewebe-Grenzfläche verläuft.

13. Chirurgisches Instrument nach Anspruch 10, **dadurch gekennzeichnet, dass** das Messer dazu eingerichtet ist, sich bei Betätigung der Betätigungseinrichtung von einem Endbereich der Greifbacken zu dem entgegengesetzten Endbereich der Greifbacken zu bewegen, allgemein entlang der Greifbacken, um das zwischen der ersten Gewebe-Grenzfläche und der zweiten Gewebe-Grenzfläche gegriffene Gewebe sequentiell zu schneiden.

14. Chirurgisches Instrument nach einem der Ansprüche 1 bis 13, **dadurch gekennzeichnet, dass** die Bewegungseinrichtung (152; 252; 352) dazu eingerichtet ist, die erste Gewebe-Grenzfläche (130; 230; 330) relativ zu der zweiten Gewebe-Grenzfläche (132; 232; 332) in einer allgemein parallelen Beziehung zu bewegen.

15. Chirurgisches Instrument nach einem der Ansprüche 1 bis 14, **dadurch gekennzeichnet, dass** der Körperbereich (310, 312) des Rahmens einen Schaft (312) aufweist und dass die Längsachsen der ersten Gewebe-Grenzfläche (330) und der zweiten Gewebe-Grenzfläche (332) quer in Bezug auf die Längsachse des Schafts (312) verlaufen.

16. Chirurgisches Instrument nach einem der Ansprüche 1 bis 14, **dadurch gekennzeichnet, dass** der Körperbereich (110, 112; 210) des Rahmens einen Schaft (112; 210) aufweist und dass die Längsachsen der ersten Gewebe-Grenzfläche (130; 230) und der zweiten Gewebe-Grenzfläche (132; 232) allgemein parallel in Bezug auf die Längsachse des Schafts (112; 210) verlaufen.

17. Chirurgisches Instrument nach einem der Ansprüche 1 bis 16, **dadurch gekennzeichnet, dass** das chirurgische Instrument ein endoskopisches Instrument (100) ist.

18. Chirurgisches Instrument nach einem der Ansprüche 1 bis 17, **dadurch gekennzeichnet, dass** die Backenanordnung (120) entfernbar im distalen Endbereich (116) des Körperbereichs (110; 112) montiert ist.

## Revendications

1. Instrument chirurgical, comportant :
- une ossature ayant une partie de corps (110, 112 ; 210 ; 310, 312) et une poignée (150 ; 210 ; 350),
- un ensemble de mâchoires (120 ; 220 ; 320) situé dans la zone distale de l'instrument (100 ; 200 ; 300), l'ensemble de mâchoires (120 ; 220 ; 320) ayant une première interface tissulaire généralement rectiligne (130 ; 230 ; 330), une seconde interface tissulaire géné-ralement rectiligne (132 ; 232 ; 332) et un effecteur tissulaire, et l'ensemble de mâchoires (120 ; 220 ; 320) étant adapté pour saisir le tissu d'un patient entre la première interface tissulaire (130 ; 230 ; 330) et la seconde interface tissulaire (132 ; 232 ; 332), et pour pé-nétrer dans le tissu saisi par l'intermédiaire de l'effecteur tissulaire,
- un dispositif de déplacement (152 ; 252 ; 352) adapté pour déplacer la première interface tissulaire (130 ; 230 ; 330) par rapport à la seconde inter-face tissulaire (132 ; 232 ; 332), et
- un dispositif d'actionnement (154 ; 254 ; 354) adapté pour actionner l'effecteur tissulaire,
- **caractérisé en ce que** la première interface tissulaire (130 ; 230 ; 330) et la seconde interface tis-sulaire (132 ; 232 ; 332), localement et en superposition sur leur rectitude générale, ont une forme généralement ondulée, les crêtes (134) et les vallées (135) de la première interface tissulaire (130 ; 230 ; 330) s'appariant généralement avec les vallées (136) et les crêtes (137), respectivement, de la seconde interface tissulaire (132 ; 232 ; 332).

2. Instrument chirurgical selon la revendication 1, **caractérisé en ce que** l'instrument chirurgical (100 ; 200 ; 300) est un instrument d'agrafage, dans le-quel l'ensemble de mâchoires (120 ; 220 ; 320) comporte un dispositif à cartouche (122 ; 222 ; 322) ayant la première interface tissulaire (130 ; 230 ; 330) et une enclume (126 ; 226 ; 326) ayant la seconde interface tissulaire (132 ; 232 ; 332), l'effecteur tissulaire comportant, associé au dispositif à cartouche (122 ; 222 ; 322), au moins un rang généralement droit d'agrafes qui, lors de l'actionnement du dispositif d'actionnement (154 ; 254 ; 354), sortent de la première interface tissulaire (130 ; 230 ; 330), la seconde interface tissulaire (132 ; 232 ; 332) étant adaptée pour former les extrémités des agrafes.

3. Instrument chirurgical selon la revendication 2, **caractérisé en ce que** la ligne définissant le rang d'agrafes suit la forme ondulée de la première interface tissulaire (130 ; 230 ; 330).

4. Instrument chirurgical selon la revendication 2 ou 3, **caractérisé en ce que** l'effecteur tissulaire comporte une lame, qui est contenue dans le dispositif à cartouche (122 ; 222 ; 322), s'étend généralement parallèlement au rang d'agrafes et, lors de l'actionnement du dispositif d'actionnement (154 ; 254 ; 354), se déplace vers l'enclume (126 ; 226 ; 326).

5. Instrument chirurgical selon la revendication 4, **caractérisé en ce que** la lame a un bord coupant ondulé qui, de préférence, s'étend généralement parallèlement a la forme ondulée de la première interface tissulaire (330).

6. Instrument chirurgical selon la revendication 2 ou 3, **caractérisé en ce que** l'effecteur tissulaire comporte une lame, qui est adaptée pour se déplacer, lors de l'acitionnement du dispositif d'actionnement (154 ; 254), à partir d'une première zone d'extrémité (128 ; 228) du dispositif à cartouche (122 ; 222) jusqu'à la zone d'extrémité opposée (140 ; 240) du dispositif à cartouche (122 ; 222), généralement parallèlement au rang d'agrafes, et le long de celui-ci, pour découper séquentiellement le tissu saisi entre la première interface tissulaire (130 ; 230) et la seconde interface tissulaire (132 ; 232).

7. Instrument chirurgical selon l'une quelconque des revendications 4 à 6, **caractérisé en ce qu'**il y a au moins un rang d'agrafes de chaque côté de la lame.

8. Instrument chirurgical selon l'une quelconque des revendications 2 à 7, **caractérisé en ce que** l'enclume est amovible.

9. Instrument chirurgical selon l'une quelconque des revendications 2 à 8, **caractérisé en ce que** le dispositif à cartouche (122 ; 222 ; 322) comporte une cartouche amovible (124) contenant les agrafes.

10. Instrument chirurgical selon la revendication 1, **caractérisé en ce que** l'instrument chirurgical est un instrument coupant, dans lequel l'ensemble de mâchoires comporte une première mâchoire de préhension ayant la première interface tissulaire et une seconde mâchoire de préhension ayant la seconde interface tissulaire, l'effecteur tissulaire comportant une lame qui est adaptée pour découper, lors de l'actionnement du dispositif d'actionnement, le tissu saisi entre la première interface tissulaire et la seconde interface tissulaire.

11. Instrument chirurgical selon la revendication 10, **caractérisé en ce que** la lame est généralement rectiligne, s'étend généralement le long de l'ensemble de mâchoires, et lors d'un actionnement du dispositif d'actionnement, se déplace à partir de la première interface tissulaire vers la seconde interface tissulaire.

12. Instrument chirurgical selon la revendication 11, **caractérisé en ce que** la lame a un bord coupant ondulé qui, de préférence, s'étend généralement parallèlement à la forme ondulée de la première interface tissulaire.

13. Instrument chirurgical selon la revendication 10, **caractérisé en ce que** la lame est adaptée pour se déplacer, lors de l'actionnement du dispositif d'actionnement, à partir d'une première zone d'extrémité des mâchoires de préhension vers la zone d'extrémité opposée des mâchoires de préhension, généralement le long des mâchoires de préhension, pour couper séquentiellement le tissu saisi entre la première interface tissulaire et la seconde interface tissulaire.

14. Instrument chirurgical selon l'une quelconque des revendications 1 à 13, **caractérisé en ce que** le dispositif de déplacement (152 ; 252 ; 352) est adapté pour déplacer la première interface tissulaire (130 ; 230 ; 330) par rapport à la seconde interface tissulaire (132 ; 232 ; 332) selon une relation généralement parallèle.

15. Instrument chirurgical selon l'une quelconque des revendications 1 à 14, **caractérisé en ce que** la partie de corps (310 , 312) de l'ossature comporte un arbre (312), et **en ce que** les axes longitudinaux de la première interface tissulaire (330) et de la seconde interface tissulaire (332) s'étendent transversalement par rapport à l'axe longitudinal de l'arbre (312).

16. Instrument chirurgical selon l'une quelconque des revendications 1 à 14, **caractérisé en ce que** la partie de corps (110 , 112 ; 210) de l'ossature comporte un arbre (112 ; 210), et **en ce que** les axes longitudinaux de la première interface tissulaire (130 ; 230) et de la seconde interface tissulaire (132 ; 232) s'étendent généralement parallèlement par rapport à l'axe longitudinal de l'arbre (112 ; 210).

17. Instrument chirurgical selon l'une quelconque des revendications 1 à 16, **caractérisé en ce que** l'instrument chirurgical est un instrument endoscopique (100).

18. Instrument chirurgical selon l'une quelconque des revendications 1 à 17, **caractérisé en ce que** l'ensemble de mâchoires (120) est monté de manière amovible dans la zone d'extrémité distale (116) de la partie de corps (110 ; 112).
